(19) Europäisches Patentamt
European Patent Office
Office européen des brevets

(11) Publication number: 0 248 999
A2

(12) # EUROPEAN PATENT APPLICATION

(21) Application number: 87105324.5

(22) Date of filing: 10.04.87

(51) Int. Cl.[3]: A 61 K 31/557
//C07C177/00

(30) Priority: 11.04.86 US 851023

(43) Date of publication of application:
16.12.87 Bulletin 87/51

(84) Designated Contracting States:
AT BE CH DE ES FR GB GR IT LI LU NL SE

(71) Applicant: SYNTEX (U.S.A.) INC.
3401 Hillview Avenue
Palo Alto, California 94304(US)

(72) Inventor: Sevelius, Hilli
58 Austin Avenue
Atherton California 94025(US)

(72) Inventor: Schwartz, Kenneth E.
511 Virginia Avenue
San Mateo California 94402(US)

(74) Representative: Barz, Peter, Dr. et al,
Patentanwälte Dipl.-Ing. G. Dannenberg Dr. P. Weinhold,
Dr. D. Gudel Dipl.-Ing. S. Schubert, Dr. P. Barz
Siegfriedstrasse 8
D-8000 München 40(DE)

(54) **Uses of enprostil-type prostaglandins in regulating fat and carbohydrate metabolism.**

(57) This invention provides an isomer, or mixture thereof, of a compound of the formula:

(dl) 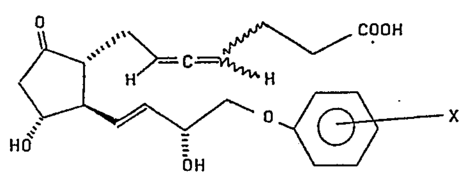

wherein X is hydrogen, halogen, trifluoromethyl, lower alkyl or lower alkoxy; one wavy line denotes the α configuration and the other β; or a pharmaceutically acceptable salt or lower alkyl ester thereof, and pharmaceutical compositions containing these compounds, said compounds and compositions being used for regulating fat or carbohydrate metabolism, in particular, for suppressing postprandial cholesterolemia, postprandial lipemia, postprandial chylomicronemia, postprandial serum glucose elevation, postprandial serum insulin elevation, postprandial serum GIP elevation, postprandial serum C-peptide elevation, reducing serum triglycerides, serum cholesterol or serum LDL-cholesterol, or treating atherosclerosis.



Croydon Printing Company Ltd.

# USES OF ENPROSTIL-TYPE PROSTAGLANDINS IN REGULATING FAT AND CARBOHYDRATE METABOLISM

This invention relates to new pharmaceutical uses of an isomer, or a mixture thereof, of compounds of the formula:

(dl) 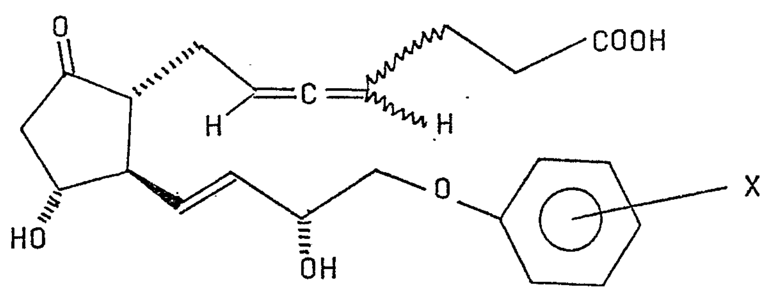

wherein X is hydrogen, halogen, trifluoromethyl, lower alkyl, or lower alkoxy; and one wavy line denotes the α configuration, and the other, β, and the pharmaceutically-acceptable salts or lower alkyl esters thereof.

U.S. Patent No. 4,459,310 shows the use of 16-(lower alkyl)-16-hydroxy-prostaglandins, such as misoprostol (USAN name), to lower cholesterol blood levels in patients with hypercholesterolemia. The dosage form is not specified in this patent, nor is mention made of LDL-cholesterol or apoproteins.

Konturek et al., Prostaglandins, 15, 591-602 (1978) reported that (15S),15-methyl-$PGE_2$ methyl ester administered by tube to the duodenum lowered insulin levels. It was noted, however, that there was no change in the serum glucose levels observed, despite lower insulin levels. Intravenous administration of (15S),15-methyl-$PGE_2$ ethyl ester similarly lowered insulin levels without affecting glucose levels. There was no change in the time to peak serum glucose levels following administration of the above-identified (15S),15-methyl-$PGE_2$ methyl ester either intraduodenally or intravenously.

A relative state of insulin resistance, associated with normal to elevated plasma insulin levels, is present in adults with non-insulin-dependent diabetes mellitus. The cause of this condition is unknown but it has been associated with excessive weight gain. A prostaglandin-induced lowering of circulating insulin levels might lead to a decrease in insulin resistance and an improvement in the degree of diabetic control. Furthermore, in many of these individuals, elevated serum insulin levels are

associated with an elevation in serum gastric inhibitory polypeptide (GIP), also known as glucose-dependent insulinotropic polypeptide, a peptide hormone found in the gastrointestinal mucosa which is widely thought to be one factor controlling the release of pancreatic insulin (Dupre et al., J.Clin.Endocrinol.Metab., 37, 826 (1973)). A reduction in serum GIP levels might also lower the degree of insulin resistance present in some individuals.

Furthermore, there is a similar surge in serum GIP levels in alimentary hypoglycemia, a condition characterized by a late surge in serum insulin following a carbohydrate meal and a state of relative hypoglycemia, (Hadji-Georgopoulos et al., J.Clin.Endocrinol.Metab., 56, 648 (1983)).

A clinical problem present in the treatment of diabetes mellitus which must be treated with insulin administered subcutaneously, Type I diabetes, is that of coordinating the peak in serum insulin levels which occurs following subcutaneous administration of insulin with the peak in serum glucose levels following a meal. There may be some delay in insulin absorption and activity with the result that excessive surges in serum glucose may occur shortly after a meal. The conventional clinical approach to this problem is to administer subcutaneously a mixture of short-acting and long-acting insulins. There is no conventional method for delaying the time to onset of peak serum glucose following a meal.

A delay in the time to peak blood glucose levels or a lowering of the peak level that is ultimately achieved may improve diabetic control in both insulin-dependent and non-insulin-dependent diabetics.

The role of GIP in the pathogenesis of non-insulin-dependent diabetes mellitus, Type II diabetes, is unclear. An elevation of GIP levels in association with

the non-insulin-dependent diabetic state has been described. "The significance of this observation is uncertain. However, since our current understanding suggests that GIP may be an important enteric signal for the release of insulin in man, . . . , GIP may play a role in the pathogenesis of diabetes mellitus" (Crockett et al., Diabetes, 25, 931-35, (1976)).

Previous methods of lowering GIP levels have included induction of weight loss, restriction of calorie intake, and administration of drugs to the patient which inhibit intestinal glucosidase activity. One such drug is Acarbose. See Proceedings of the First International Symposium on Acarbose, 1982, Excerpta Medica, Princeton, New Jersey. Acarbose reduces postprandial serum glucose and insulin levels as well as postprandial GIP levels. There is no delay in the time to achieve peak glucose or insulin levels following a meal, however, nor is there a change in total serum cholesterol. An improvement in the condition of patients with diabetes mellitus has been observed, however. This finding may be related to an inhibition of metabolism of complex sugars in the brush border in the intestinal lumen.

Both Type I and Type II diabetics are predisposed to premature atherosclerosis and cardiovascular disease. Certain other individuals show abnormal glucose tolerance following a meal, but are not overtly diabetic. These individuals may also have greater than average risk of these diseases, particularly if hypercholesterolemia is present.

That hypercholesterolemia and dietary cholesterol intake are related to atherosclerosis and coronary artery disease is now firmly established (Stamler, J.Prog.Biochem.Pharmacol., 19, 245-303 (1983); Blankenhorn, N.Engl.J.Med., 312, 851-2 (1985); Kushi et al., N.Engl.J.Med., 312, 811-7 (1985)). Massive

documentation in the medical literature has demonstrated both in human and primate models that severe atherosclerosis is the underlying pathologic process in most cases of clinical coronary disease. Sustained hypercholesterolemic hyperlipidemia, regardless of cause, is associated with frequent, premature, severe atherosclerotic coronary heart disease (CHD). In a wide range of experimental animal models, sustained ingestion of diets containing increased quantities of cholesterol and fat is a virtual prerequisite for production of significant atherosclerosis (Stamler, op. cit.). Furthermore, it is now well-recognized that in populations where the mean cholesterol levels are low, clinical CHD and severe coronary atherosclerosis at postmortem are rare. In populations studied prospectively, risk of premature atherosclerotic disease is increased in the presence of hypercholesterolemia and hyperlipidemia (NIH Consensus Development Conference Statement, Arteriosclerosis, 5, 505-12 (1985)).

The Framingham Study has shown that a relatively large amount of cholesterol in the low-density lipoprotein fraction of serum cholesterol (LDL-cholesterol) is especially atherogenic, whereas that in the high-density fraction (HDL-cholesterol) appears protective (Kannel et al., Ann.Int.Med., 40, 85-91 (1979)). Attempts at lowering atherogenic lipoproteins through pharmacologic and dietary intervention have conclusively shown a reduction in the incidence of coronary heart disease (Lipid Research Clinics Program, J.Am.Med.Assn., 251, 351-64 (1984)).

The above effects can be noted in the absence of hypercholesterolemia. Data in animal models have shown dietary intake of cholesterol and saturated fats in amounts lower than that required to elevate serum cholesterol is also atherogenic and will elevate LDL-cholesterol and other lipoproteins (Armstrong, Circ.Res., 34, 447-54 (1974)). Seventy percent (70%) of plasma and serum cholesterol circulates bound to LDL, and LDL is the key atherogenic lipoprotein fraction (Stamler, op. cit.).

Dietary intake of cholesterol and saturated fats is central to most arguments regarding the pathogenesis of atherosclerosis and CHD in the majority of individuals in Western society (Stamler, op. cit.; NIH Consensus Development Conference Statement, op. cit.).

Dietary fat and cholesterol are packaged in the core of chylomicrons and are transported by the lymphatic system into the blood. During active fat absorption, the number and size of these particles increases appreciably, and both plasma and serum take on an opalescent appearance after a large, fatty meal.

Current methods for treatment of hypercholesterolemia and established atherosclerosis place major emphasis on dietary manipulation with reduction of cholesterol intake and saturated fatty acids in the diet, and indeed partial regression of established atherosclerosis has been demonstrated both in human and nonhuman primate models with dietary manipulation (Arntzenius et al, N.Engl.J.Med., 312, 805-11 (1985)).

Dietary intake of cholesterol and saturated fats is central to most arguments regarding the pathogenesis of atherosclerosis and coronary artery disease (Stamler, op. cit.; NIH Consensus Development Conference Statement, op. cit.), as these substances are the major factors influencing plasma lipoprotein levels (Havel et al., Ann.Rev.Med., 33, 417-33 (1982)). Although the mechanisms behind these effects are not fully understood, it is well-recognized that dietary intake of both cholesterol and saturated fats mainly increases the plasma concentrations of LDL and VLDL (very low density lipoprotein) particles and their cholesterol content

(Havel et al., in "Metabolic Control and Disease", ed. Bondy et al., 8th ed., 393-494, W.B. Saunders Co., Philadelphia). VLDL particles are in turn metabolised still further to LDL particles, and hence a diet high in saturated fats and cholesterol is particularly atherogenic (Stamler, op. cit.).

Current methods for treatment of hypercholesterolemia and established atherosclerosis thus place major emphasis on dietary manipulation with reduction of dietary cholesterol and saturated fatty acids. Indeed, a decrease in progression of established atherosclerosis has been recently documented in human and subhuman primate models following these dietary alterations (Arntzenius et al., op. cit.).

Lipoproteins are spherical particles that transport non-polar (water-insoluble) lipids in their core. Dietary fat and cholesterol are transported as triglycerides and cholesteryl esters, respectively, in chylomicrons, the largest and least dense particles, and are transported by the lymphatic system into the blood. During active fat absorption, i.e. following a large, fatty meal, the number and size of these particles increases appreciably, and both plasma and serum take on an opalescent appearance.

A portion of the triglyceride content of the chylomicron is hydrolyzed at the surface of a variety of tissues, most notably fat and muscle cells, by an enzyme called lipoprotein lipase. The fatty acids liberated from triglyceride by this process, so-called free fatty acids, are free4 to diffuse into the adjacent tissues and are stored for later utilization (i.e. fat). The remainder of the chylomicron, now triglyceride poor and cholesterol rich, is known as a chylomicron remnant. Cholesterol taken up by the liver in chylomicron remmants may be resecreted in VLDL which is in turn converted to

other lipoproteins, especially LDL (Havel et al., Ann.Rev.Med., 33, 417-33 (1982)).

Thus, although the precise mechanisms behind the induction of atherosclerosis are still under investigation, specific dietary influences play a major role in its cause and interventions, be gastrointestinal uptake and absorption of saturated fats, unsaturated fats, and cholesterol would be expected to provide a major reduction in atherosclerosis.

Enprostil, particularly its R allenic isomer, and similar enprostil-type prostaglandins, have been shown to be potent gastric secretory inhibitors, and are effective for the treatment of ulcers and ulcerogenic conditions. Typical dosages are from 70 to 140 µg/day for these conditions.

This invention provides an isomer, or mixture thereof, of a compound of the formula:

(dl)

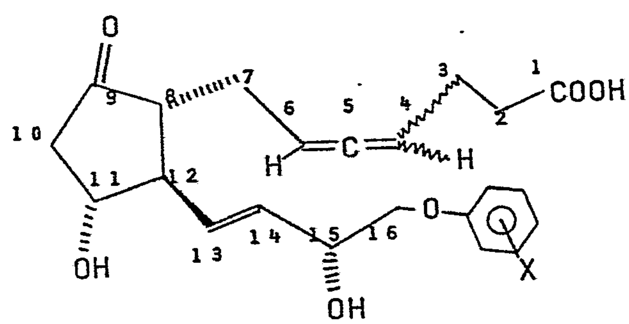

(I)

wherein X is hydrogen, halogen, trifluoromethyl, lower alkyl or lower alkoxy; one wavy line denotes the α configuration and the other β; or a pharmaceutically acceptable salt or lower alkyl ester thereof,

for regulating fat or carbohydrate metabolism, in particular, for suppressing postprandial cholesterolemia,

postprandial lipemia, postprandial chylomicronemia, postprandial serum glucose elevation, postprandial serum insulin elevation, postprandial serum GIP elevation, postprandial serum C-peptide elevation, reducing serum triglycerides, serum cholesterol or serum LDL-cholesterol, or treating atherosclerosis.

The compounds can thus be used to treat a variety of conditions where regulation of fat or carbohydrate metabolism, i.e., controlling levels of serum cholesterol in normal subjects (normocholesterolemic), triglycerides, chylomicrons, insulin or GIP would be beneficial. Such conditions include atherosclerosis, hypercholesterolemia, hypertriglyceridemia, conditions characterized by hyperchylomicronemia and similar conditions, insulin-dependent and non-insulin-dependent diabetes mellitus, reactive hypoglycemia, pancreatic cell hyperplasia (nesidioblastosis), and similar conditions.

This invention also provides suitable compositions containing the above compounds.

Definitions:

Unless the context otherwise requires, the following terms used in the specification and claims have the meanings given below:

"halogen" refers to fluorine, chlorine, bromine, or iodine.

"lower alkyl" refers to straight or branched saturated hydrocarbon radicals having from one to four carbon atoms, e.g., methyl, ethyl, isopropyl, t-butyl, and the like. A preferred lower alkyl is methyl.

"lower alkoxy" refers to radicals of the form -OR, in which R is "lower alkyl" as defined above.

"pharmaceutically-acceptable salt" refers to salts of the compound of formula I which possess the desired pharmacological activity and which are neither biologically or otherwise undesirable. These salts are prepared from either inorganic or organic bases. Salts derived from inorganic bases include the sodium, potassium, lithium, ammonium, calcium, magnesium, ferrous, zinc, copper, manganous, aluminum, ferric, manganic salts, and the like. Preferred inorganic salts are the ammonium, sodium, potassium, calcium, and magnesium salts. Salts derived from organic bases include salts of primary, secondary, and tertiary amines, substituted amines including naturally-occurring substituted amines, and cyclic amines, including isopropylamine, trimethylamine, diethylamine, triethylamine, tripropylamine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, N-ethylpiperidine, and the like. Preferred organic bases

are isopropylamine, diethylamine, ethanolamine, piperidine, tromethamine, choline, and caffeine.

"Suppressing" a symptom or condition includes one or both of:

(i) reducing the severity of that symptom or condition; or

(ii) delaying the onset and/or peak of that symptom or condition.

"Treatment" refers to any treatment of a disease (or a symptom or condition thereof) in a mammal, particularly a human, and includes one or more of:

(i) preventing the disease from occurring in a subject which may be predisposed to the disease but has not yet been diagnosed as having it;

(ii) inhibiting the disease, i.e., arresting its development; or

(iii) relieving the disease, i.e., causing regression of the disease.

"Normocholesterolemic subject" refers to a mammal, in particular a human who has serum cholesterol under 250 mg/dl.

Isomerism and Synthesis of the Compounds of Formula (I):

The compounds of formula (I) possess asymmetric centers, and thus can be produced as racemic or non-racemic mixtures, or as individual (+)- or (-)-enantiomers. The individual enantiomers may be obtained by resolving a racemic or non-racemic mixture of an intermediate at some appropriate stage of the synthesis. It is understood that the racemic or non-racemic mixtures and the individual (+)- or (-)-enantiomers are encompassed within the scope of this invention.

Formula (I) includes any single structure below (Ia, Ia', Ib, and Ib'), all permutations of two or three components in any proportions, and mixtures of all four components in any proportions.

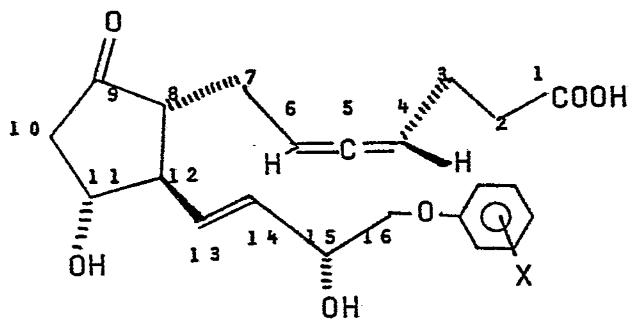

(Ia)

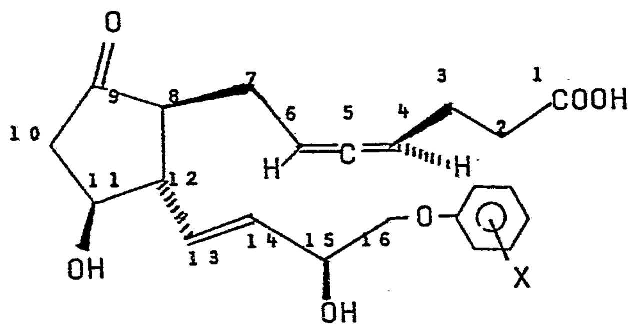

(Ia')

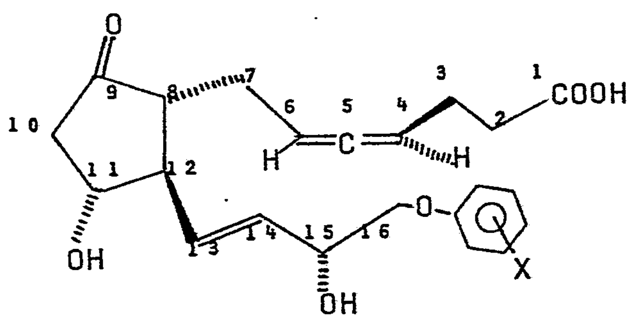

(Ib)

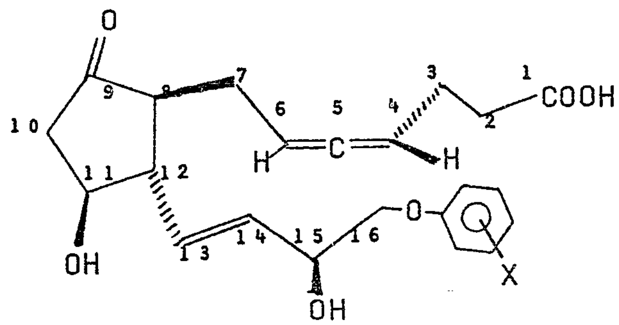

(Ib')

For the sake of simplicity only one enantiomer, i.e. the enantiomer having the natural prostaglandin configuration, will be further depicted, but such depiction is not to be construed as limiting unless the context otherwise requires. The natural configurations are represented by formulae (Ia) and (Ib).

Compounds of formula (I) may readily be prepared by those of ordinary skill in the art using methods described in the scientific and patent literature. For example, the compounds of formula (I) are disclosed in U.S. Patent No. 4,178,457, as are their preparation from the corresponding 9-hydroxy-prostaglandins (their PGF analogs). The PGF analogs of the compounds of formula (I) are disclosed in U.S. Patent No. 3,985,791, as are their preparation from starting materials known to the art. Both of these references disclose the preparation of the natural, i.e. 8α,11α,15α-configuration, materials, their corresponding (8R)-antimers, and mixtures, particularly racemic mixtures, thereof. The four individual stereoisomers of formula (I), i.e. formulae (Ia), (Ia'), (Ib), and (Ib'), are disclosed in allowed U.S. Patent Application Serial No. 06/564,386 and European Published Application No. 0 146 935, as are their preparation from starting materials known to the art. The compounds and procedures set forth in the patents and patent applications referred to in this paragraph are incorporated herein by reference.

A preferred compound of the present invention is enprostil (USAN name), the methyl ester of the compound of formula (I) wherein X is hydrogen, i.e. methyl (±)-7-[(1R*,2R*,3R*)-3-hydroxy-2-[(E)-(3R*)-3-hydroxy-4-phenoxy-1-butenyl]-5-oxocyclopentyl]-4,5-heptadienoate, also known as methyl (±)-9-oxo-11α,15α-dihydroxy-16-phenoxy-17,18,19,20-tetranorprosta-4,5,13(E)-trienoate. Enprostil, as with the other compounds of formula (I), is a mixture of four isomers, and a particularly preferred compound of the present invention is the R allenic isomer of enprostil, the methyl ester of the compound of formula (Ia) wherein X is hydrogen, i.e. methyl (4,5,6R, 8R)-9-oxo-11α,15α-dihydroxy-16-phenoxy-17,18,19,20-tetranorprosta-4,5,13(E)-trienoate. This isomer has been shown to occur in crystalline form, thereby facilitating handling, formulation into solid dosage forms, and analysis, and improving chemical stability, and displays improved biological properties (e.g. increased potency and lower toxicity) in animal antisecretory assays.

Administration and Formulation:

Another aspect of the present invention relates to pharmaceutical compositions comprising a therapeutically effective amount of a compound of formula (I), or a pharmaceutically-acceptable salt or lower alkyl ester thereof, in admixture with a pharmaceutically-acceptable carrier. A therapeutically effective amount is that amount which, when administered to a mammal in need thereof, is sufficient to effect treatment, as defined above. Thus, the level of the drug in the formulation can vary from about 0.001 percent weight (%w) to about 10%w of the drug based on the total formulation and about 99.999%w to 90%w excipient. Preferably the drug is present at a level of about 0.01%w to about 0.5%w.

Useful pharmaceutical carriers for the preparation of the pharmaceutical compositions hereof can be solids or liquids. Thus, the compositions can take the form of tablets, pills, capsules, powders, sustained release formulations, solutions, suspensions, elixirs, and the like. Carriers can be selected from the various oils, including those of petroleum, animal, vegetable or synthetic origin, for example, peanut oil, soybean oil, mineral oil, sesame oil, and the like, water, saline, aqueous dextrose, glycols, and the like. Suitable solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol, and the like. Other suitable pharmaceutical carriers and their formulations are described in "Remington's Pharmaceutical Sciences", 16 ed., ed. Osol et al., Mack Publishing Co., Easton, Pennsylvania (1980).

In the practice of the above described method of the present invention a therapeutically effective amount of a compound of formula (I) or a pharmaceutical composition containing same is administered via any of the usual and acceptable methods known in the art, either singly or in combination with another compound or compounds of the present invention or other pharmaceutical agents. These compounds or compositions can thus be administered orally, systemically (e.g., transdermally, intranasally, or by suppository) or parenterally (e.g., intramuscularly, subcutaneously, or intravenously), and can be administered either in the form of solid or liquid dosages including tablets, solutions, suspensions, aerosols, and the like, as discussed in more detail above. It is preferred to administer compounds of formula (I) orally.

Particularly preferred oral formulations of the compounds of formula (I), especially of enprostil, and more especially of the R allenic isomer thereof, are formulations in which the compound is dissolved in a propylene glycol diester of a short-chain fatty acid or in a cyclic carbonate diester, such as propylene carbonate. For a solid dosage form, the solution, e.g. in propylene carbonate, is preferably encapsulated in a soft-shelled gelatine capsule. Such diester solutions, and the preparation and encapsulation thereof, are disclosed in U.S. Patents Nos. 4,328,245; 4,409,239; and 4,410,545, which are incorporated herein by reference. For a liquid dosage form, the solution, e.g. in propylene carbonate, may be diluted with a sufficient quantity of a pharmaceutically-acceptable liquid carrier, e.g. water, to be easily measured for administration.

Other useful formulations include those set forth in U.S. Patents Nos. Re. 28,819 and 4,358,603, both of which are incorporated herein by reference.

The formulation can be administered in a single unit dosage form for continuous treatment or in a single unit dosage form ad libitum when relief of symptoms is specifically required.

Suitable dosages of the compounds of formula (I) for the methods disclosed in this application are less than 100 µg/day (for the "average" 70 Kg human, with corresponding variation for mammals of different weights, as appropriate), usually between 4 and 80 µg/day. The compound may be administered as a single dose, preferably h.s. (before sleeping), or in multiple doses. If multiple doses are given, they are preferably given preprandially, before at least the two main meals (BID dosing), all three meals (TID dosing), or before the meals and h.s. (QID dosing). Suitable TID or QID doses are thus between 1 and 20 µg; and suitable pharmaceutical compositions of this

invention thus contain between 1 and 20 μg of a compound of formula (I) in admixture with a suitable pharmaceutical carrier.

Suitable doses of enprostil are as discussed above with reference to the compounds of formula (I) in general, and will usually be in the upper two-thirds, preferably the middle one-third, of the range disclosed. Suitable doses of the R allenic isomer of enprostil are in the lower one-third of the range disclosed above with respect to the compounds of formula (I) in general.

An example of a suitable composition of a 10 μg unit dose form for enprostil in this invention is:

| | |
|---|---|
| Enprostil | 10 μg |
| Propylene carbonate | q.s. to 0.28 ml |

The resulting solution is encapsulated in a #5 soft elastic gelatine capsule.

For the methods of regulating fat and carbohydrate metabolism of this invention, it is desirable (in contrast to the treatment of ulcers or ulcerogenic conditions) that the compound of formula (I) be released to the small intestine rather than to the stomach, and dosage forms which deliver the compound to the small intestine are preferred for that reason. Such forms are well-known to the pharmaceutical art, and include enteric-coated tablets, capsules, and the like. If such enteric-coated forms are used for the formulations of this invention, the doses of the compounds of formula (I), enprostil, or the R allenic isomer of enprostil, will preferably be at the lower end of the ranges for each disclosed above.

Thus, the compounds of formula (I), enprostil, or the R allenic isomer of enprostil, are effective for the methods of this invention at doses below those currently

used or believed effective for the treatment of ulcers or ulcerogenic conditions.

While not wishing to be bound by any theory of action of the compounds of formula (I), the primary rationale for the treatment of atherosclerosis by the method of this invention is believed to be the suppression of cholesterol and fatty acid absorption. However, other mechanisms may also be active. In particular, interactions with cholesterol esterase, HMG-CoA reductase, and lipoprotein lipase may affect the formation of cholesteryl ester, synthesis of endogenous cholesterol, or chylomicron metabolism and clearance.

The compounds of formula (I) are thus effective in methods of suppressing postprandial cholesterolemia, lipemia, chylomicronemia, serum glucose elevation, serum insulin elevation (including lowering serum insulin levels without raising blood glucose), serum GIP elevation, or serum C-peptide elevation; or reducing serum triglycerides, serum cholesterol, or serum LDL-cholesterol. The compounds can thus be used to treat a variety of conditions where regulation of fat or carbohydrate metabolism, i.e. controlling levels of serum cholesterol, triglycerides, chylomicrons, insulin, or GIP would be beneficial. Such conditions include atherosclerosis, hypercholesterolemia, hypertriglyceridemia, conditions characterized by hyperchylomicronemia, and similar conditions, insulin-dependent and non-insulin-dependent diabetes mellitus, reactive hypoglycemia, pancreatic cell hyperplasia (nesidioblastosis), and similar conditions.

## EXAMPLES

The following non-limiting examples will further clarify the nature of this invention. In these examples, serum lipids and cholesterol were measured by standard methods ("Manual of Laboratory Operations, Lipid Research Clinic Program, Lipid and Lipoprotein Analysis", DHEW Publication No. (NIH) 75-628 (1974)); serum insulin, GIP, and C-peptide were measured by radioimmunoassay; and apoproteins were measured by radial immunodiffusion.

### Example I

This example shows the effect of orally administered enprostil on serum cholesterol.

A group of 20 patients with high cholesterol baseline were orally given 70 µg enprostil twice daily. After five weeks of administration eight showed no change, eleven showed greater than 10 percent reduction,and one showed greater than 25 percent reduction. The median change for the group was 16 percent reduction, with 60 percent of the patients tested showing a significant reduction in serum cholesterol.

A second group of 16 patients with a high cholesterol baseline were given 35 µg enprostil twice daily. After five weeks of administration seven showed no change, eight showed greater than 10 percent serum cholesterol reduction and one showed greater than 25 percent reduction. The median change was eleven percent reduction and 56 percent the patients showed serum cholesterol reduction.

A third group of 13 individuals were given a placebo. After five weeks one showed an increase in cholesterol, and eleven showed no change, and one showed a 10 percent decrease. The median change was a 2 percent increase in cholesterol.

Example 2

This example shows the effect of orally administered enprostil on serum cholesterol levels.

Table 1 shows the results of a study of sixtyfive hypercholesterolemic patients (that is serum cholesterol above 250 µg/dl) given enprostil in doses ranging from 7 µg BID to 70 µg BID to reduce cholesterol compared to 27 subjects who received placebo.

TABLE 1

| | Amount of enprostil administered BID: | | | |
|---|---|---|---|---|
| | Placebo | 70 µg | 35 µg | 7 µg |
| Cholesterol % decrease | 15% | 64% | 64% | 67% |
| (combined 2-12 week of dosing) | | | | |
| Median % change | 0% | -17% | -13% | -11% |
| Number of subjects | 27 | 28 | 28 | 9 |

To further clarify the nature of these changes in a prospective manner, normal subjects were dosed with enprostil for periods ranging from 8 days up to 44 days in duration.

In a 9-day placebo-controlled cross-over study, with each subject serving as his own control, the mean reduction in cholesterol, LDL-cholesterol, and HDL-cholesterol are shown in Table 2. There was essentially no change in total triglycerides.

TABLE 2

| | CONTROL | ENPROSTIL |
|---|---|---|
| Mean reduction of cholesterol | -1.2% | -15.0% |
| Mean reduction of LDL-cholesterol | +1.7% | -21.0% |
| Mean reduction of HDL-cholesterol | -7.8% | -9.2% |
| Mean reduction of apoprotein-A | -5.3% | -1.8% |
| Mean reduction of apoprotein-B | -1.6 | -16.0% |

These findings are surprising since the subjects in this study were normocholesterolemic at baseline (that is serum cholesterol under 250 mg/dl) and were on a free diet with no attempt to regulate dietary cholesterol intake.

Elevated total serum cholesterol levels and LDL-cholesterol levels are known to be atherogenic. It is known that for every 1% reduction in cholesterol there may be as much as a 2% reduction in myocardial morbidity and mortality (Lipid Research Clinics Program, J.Am.Med.Assn., 251, 365-374 (1984)). It appears that adverse cardiac events are reduced by reduction in total cholesterol as well as LDL-cholesterol in an American population.

This example is of further significance in that it showed that enprostil is significantly affecting the apoprotein-B component of the lipoprotein molecule. Apoprotein-B is primarily associated with LDL-cholesterol as a protein carrier.

Example 3

This example shows the effect of orally administered enprostil on serum cholesterol levels in a long term study.

In a 44-day dosing study, 20 subjects participated in a placebo-controlled trial. Ten subjects received enprostil and the remainder received placebo. Data were obtained from 9 of the 10 subjects who received enprostil. Six of these nine subjects showed a reduction of total cholesterol by 16 days. Three of the individuals demonstrated a reduction in total and LDL-cholesterol throughout the entire 44-day period. In the most dramatic example, total serum cholesterol was reduced in one subject from a mean baseline of 208 mg/dl to 123 mg/dl at day 44, a 43% reduction. Similarly the subject showed a 46% reduction in LDL-cholesterol from 140 mg/dl to 75 mg/dl. This subject was normocholesterolemic at baseline and no dietary manipulation was attempted.

Example 4

This example shows the effect of orally administered enprostil on serum GIP levels. Serum GIP levels are known to increase after meals.

Groups of 10 subjects participated in a double-blind, cross-over study. Either a placebo or 70 μg enprostil was orally given 30 minutes before meal time and levels of GIP were measured. 60 minutes after the meal stimulus the recipients of the placebo showed a four-fold increase in serum GIP. However, subjects given enprostil showed no increase in GIP 60 minutes after the meal stimulus.

Example 5

This example shows the effect of orally administered enprostil on serum insulin and glucose levels.

A group of 10 subjects participated in a double-blind cross-over study. Either a placebo or enprostil was orally given 30 minutes before meal time and levels of insulin and glucose were measured after the meal.

Insulin levels increased five-fold in placebo subjects, but only two-fold in subjects receiving enprostil. In these same subjects there was no statistically or clinically significant raise in glucose levels in either group of patients. Therefore, enprostil held the levels of glucose at normal while lowering insulin levels.

Example 6

This example shows the effect of orally administered enprostil on serum insulin and glucose levels.

Eight healthy male subjects received either 70 µg enprostil per day or placebo for eight days followed by a washout period of one week and a subsequent second eight-day administration during which they received the opposite drug regime.

On day 1 and day 8 of each study period the subjects reported to the clinical studies unit fasting and were administered 70 µg of enprostil orally in a glass of water. Thirty minutes later they were administered a standard breakfast and blood samples were drawn at -30 (just prior to enprostil administration), 0 (at the time of enprostil administration), 15, 30, 60, 90, 120 and 180 minutes.

Peak serum glucose was measured between 30 and 60 minutes during the placebo phase. This was delayed to

between 60 and 90 minutes while the subjects received enprostil. Furthermore, the area under the serum glucose by time curve was reduced by approximately 15-20% compared to the placebo phase. These findings were present on both day 1 and day 8 of administration during each study phase.

Accompanying these findings was a marked reduction in serum insulin concentrations for each time point present on both day 1 and 8 as well as essentially complete inhibition of serum GIP for both study days following enprostil administration.

Example 7

This example shows the effect of orally administered enprostil on serum carbohydrate levels.

10 normal subjects participated in a 10-day placebo-controlled cross-over study, receiving either placebo or 70 µg enprostil per day. On day 1 and day 9 of each study phase they orally received 5 g of d-xylose, a sugar not normally metabolized in man. Blood measurements for serum xylose were made at -30, 0, +30, 60, 120, 180, 240, and 300 minutes following ingestion of xylose. On day 2 and day 10 of each study phase, the subjects received a 25 g intravenous glucose tolerance test, 30 minutes following enprostil administration.

There was a marked suppression and delay to peak serum xylose levels on both days 1 and 9 of enprostil administration. This was accompanied by a 30% reduction in the amount of xylose excreted over 5 hours: 1.3 g versus 1.8g.

There were no marked differences in the serum glucose by time curves following intravenous glucose administration to subjects between the placebo and the enprostil groups.

Enprostil both delays and retards the absorption of carbohydrates. This is a nonspecific effect since d-xylose is essentially a non-metabolizable sugar and is excreted intact in the human. The ability to suppress rapid absorption of glucose may facilitate improved metabolism of sugar by the liver as well as peripheral utilization with improvement and control of blood sugar profiles in diabetics who are both requiring insulin as well those who require oral pills for a regulation of the diabetes mellitus.

Example 8

This example shows the effect of orally administered enprostil on serum glucose levels.

20 subjects participated in a study lasting up to 44 days. On day -8, all 20 subjects received a placebo capsule, followed 30 minutes later by a standard breakfast with serial blood measurements. Eight days later all 20 subjects were randomized to either enprostil 35 µg BID or placebo, and continued dosing for a period of 30 days. On days 1, 8, and 30, they were given enprostil followed 30 minutes later by a standard breakfast with serial blood measurements.

Mean serum glucose curves were modified in the subjects receiving enprostil. There was a delay from approximately 60 minutes to 90 minutes in the appearance of peak blood glucose levels, which were substantially reduced from the peak blood glucose curves which were measured either in those subjects who received placebo throughout the entire study or in the active control group during the placebo dosing on day -8.

This further confirms that enprostil suppresses and delays the absorption and utilization of carbohydrates, particularly glucose.

Example 9

Six male subjects participated in a double-blind cross-over study. Either placebo or enprostil 35 µg was orally given 30 minutes before a fatty meal consisting of approximately 600 Kcal, 90% of which was fat. Following the meal, serum triglycerides and total serum cholesterol were measured at serial time points before and after removal of serum chylomicrons by ultracentrifugation. Serum HDL-cholesterol was measured only before chylomicron removal.

Before chylomicron removal, total postprandial serum triglycerides in the placebo-treatment phase increased from a mean fasting level of 86 mg/dl to a maximal mean level for the group of 160 mg/dl at 120 minutes following the meal. This contrasted with a similar fasting baseline measurement of 83 mg/dl with a very modest postprandial rise to only 105 mg/dl at 360 minutes following enprostil treatment.

Serum triglycerides were remeasured on all serum samples following ultracentrifugation to remove chylomicrons, the major carriers of postprandial triglyceride content. Following their removal, the remaining triglyceride content, primarily a reflection of VLDL-triglyceride, again showed suppression of the postprandial triglyceride rise following enprostil treatment. Fasting levels for both groups were similar, 82 and 80 mg/dl for placebo and enprostil treatments, respectively. The maximal measured postprandial rise following placebo and enprostil treatments again showed enprostil-related suppression with a maximal placebo treatment level of 115 mg/dl (180 minutes) while that for enprostil was only 89 mg/dl (360 minutes).

The difference between triglyceride measurements before and after removal of chylomicrons is mostly

attributable to the chylomicron triglyceride content. The results of the above study show virtually complete suppression of chylomicron triglyceride content following enprostil with only a modest 16 mg/dl mean rise noted at 360 minutes following the fatty meal. This contrasted to a mean rise of 46 mg/dl (120 minutes) over the fasting level following the placebo treatment.

In contrast to the usual cloudy appearance of serum following a meal, an indication of chylomicronemia, postprandial serum from subjects who received enprostil remained clear. Furthermore, following a single dose of placebo, maximum measured postprandial chylomicron triglyceride concentration was 46 mg/dl at 120 minutes. Following enprostil treatment, there was complete absence of chylomicron-associated triglyceridemia through 120 minutes, and from 180 minutes through 360 minutes, the mean chylomicron triglyceride contribution to the total serum triglyceride level was only between 5 and 16 mg/dl.

Following the meal, insulin levels were significantly lower at 30, 60, and 90 minutes following enprostil, while GIP levels were virtually completely suppressed ($p<0.05$) up to 360 minutes.

Both insulin and GIP are ordinarily secreted in response to a fat meal as nutrients (particularly fat and carbohydrate) cross the intestinal mucosa and are absorbed, and suppression, as noted above, is consistent with decreased fat absorption.

Example 10

Fortyfive New Zealand white rabbits were allocated to three groups, A, B, and C, each group consisting of 15 rabbits. Group A was fed a 2% cholesterol diet and received vehicle administered by constant oral infusion at 62 μl/day using an Alza minipump. Group B was also

fed a 2% cholesterol diet but, via the minipump, received enprostil at an infusion rate of 10 µg/Kg/day. Group C was fed a normal diet and received no treatment.

At the end of 56 days of the above treatments, rabbits were sacrificed and their aortic tissue was subjected to pathologic examination. In Group C, the placebo control group, the percent of aortic area covered by atherosclerotic plaque was under 5%. In the rabbits from Group A, which had received the high cholesterol diet with vehicle infusion, 25% of the aortic area was covered with atherosclerotic plaque. These findings contrasted with those for the Group B rabbits, which in addition to the high cholesterol diet had received enprostil by continuous infusion . In these animals, only 10% of aortic tissue was covered with atheromatous plaque. This finding is statistically significant from the Group A rabbits.

CLAIMS:

1. An isomer, or mixture thereof, of a compound of the formula:

(dl)

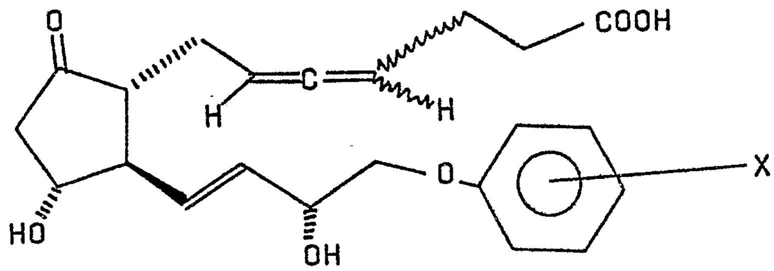

wherein X is hydrogen, halogen, trifluoromethyl, lower alkyl or lower alkoxy; one wavy line denotes the α configuration and the other β; or a pharmaceutically acceptable salt or lower alkyl ester thereof,

for regulating fat or carbohydrate metabolism, in particular, for suppressing postprandial cholesterolemia, postprandial lipemia, postprandial chylomicronemia, postprandial serum glucose elevation, postprandial serum insulin elevation, postprandial serum GIP elevation, postprandial serum C-peptide elevation, reducing serum triglycerides, serum cholesterol or serum LDL-cholesterol, or treating atherosclerosis.

2. The use of an isomer, or mixture thereof, of a compound of the formula:

(dl)

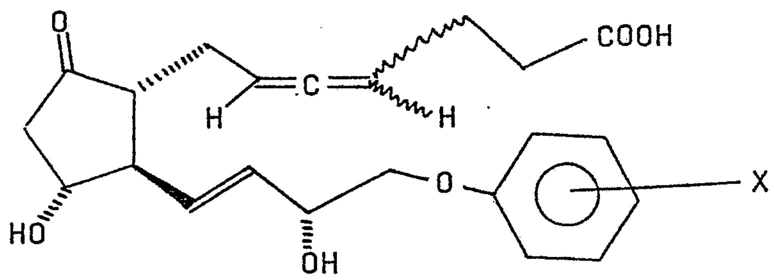

wherein X is hydrogen, halogen, trifluoromethyl, lower alkyl or lower alkoxy; one wavy line denotes the α

configuration and the other ß; or a pharmaceutically acceptable salt or lower alkyl ester thereof,

for the manufacture of a medicament for regulating fat or carbohydrate metabolism, in particular, for suppressing postprandial cholesterolemia, postprandial lipemia, postprandial chylomicronemia, postprandial serum glucose elevation, postprandial serum insulin elevation, postprandial serum GIP elevation, postprandial serum C-peptide elevation, reducing serum triglycerides, serum cholesterol or serum LDL-cholesterol, or treating atherosclerosis.

3. The use of claim 2, wherein the compound is enprostil, preferably the R allenic isomer of enprostil.

4. A pharmaceutical composition for regulating fat or carbohydrate metabolism, in particular, for suppressing postprandial cholesterolemia, postprandial lipemia, postprandial chylomicronemia, postprandial serum glucose elevation, postprandial serum insulin elevation, postprandial serum GIP elevation, postprandial serum C-peptide elevation, reducing serum triglycerides, serum cholesterol or serum LDL-cholesterol, or treating atherosclerosis, comprising between 1 and 20 μg of an isomer, or mixtures thereof, of a compound of the formula:

(dl)

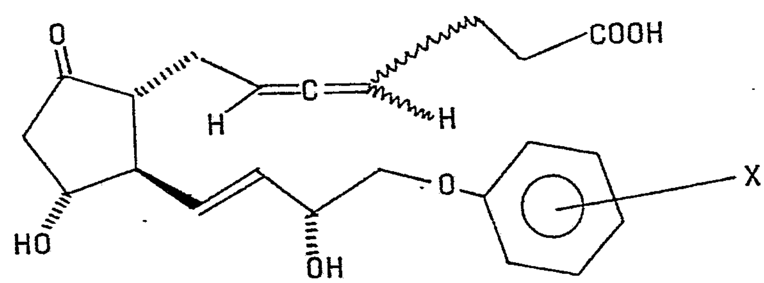

wherein X is hydrogen, halogen, trifluoromethyl, lower alkyl or lower alkoxy; one wavy line denotes the α configuration and the other ß; or a pharmaceutically

acceptable salt or lower alkyl ester thereof, in admixture with a pharmaceutically acceptable carrier.

5. The composition of claim 4, wherein the compound is enprostil, preferably the R allenic isomer of enprostil.

6. The composition of claim 4 or 5, wherein the pharmaceutical carrier comprises a cyclic carbonate diester.

7. The composition of any one of claims 4 to 6, wherein the pharmaceutical carrier includes a capsule.

8. The composition of any one of claims 4 to 6, wherein the pharmaceutical carrier includes an enteric coating.